# EUROPÄISCHE PATENTSCHRIFT

(11) **EP 2 451 357 B1**
(45) Veröffentlichungstag und Bekanntmachung des Hinweises auf die Patenterteilung: **06.01.2016**
(21) Anmeldenummer: 10724481.6
(22) Anmeldetag: 02.06.2010
(51) Int. Cl.: A61B 6/10, A61N 5/10, G21F 3/00

(54) **GEWEBEPROTEKTOR**
TISSUE PROTECTOR
PROTECTEUR TISSULAIRE

(30) Priorität: 07.09.2009 EP 09169625
(43) Veröffentlichungstag der Anmeldung: 16.05.2012
(73) Patentinhaber: HIBRAND INDUSTRIES AG, 9491 Ruggell (LI)
(72) Erfinder: BETTEGA, Remo, CH-9444 Diepoldsau (CH)
(74) Vertreter: Winter, Brandl, Fürniss, Hübner, Röss, Kaiser, Polte - Partnerschaft mbB
(86) Internationale Anmeldenummer: PCT/EP2010/057751
(87) Internationale Veröffentlichungsnummer: WO 2011/026662

(56) Entgegenhaltungen:
- EP-A1- 2 215 967
- WO-A-98/01865
- WO-A1-2009/091354
- US-A- 4 223 229
- US-A1- 2003 178 028

## Beschreibung

Die vorliegende Erfindung betrifft einen Gewebeprotektor zur Verhinderung einer Schädigung von gesundem Gewebe in unmittelbarer Umgebung eines Tumors durch Bestrahlung im Rahmen einer Tele- oder Brachytherapie, d. h. einer Tumortherapie, bei der eine geeignete Strahlung von außen auf den Körper des Patienten einwirkt bzw. bei der sich die Strahlenquelle im oder direkt am Körper befindet. Die Erfindung betrifft insbesondere einen Gewebeprotektor zur Verhinderung einer Schädigung von gesundem Gewebe in der Mundhöhle und im mittleren Rachen.

Die Möglichkeiten zum Schutz des gesunden Gewebes hängen davon ab, ob es sich um eine Teletherapie, auch externe Strahlentherapie genannt, oder eine Brachytherapie, auch interne Strahlentherapie genannt, handelt.

Zum Beispiel ist zur teletherapeutischen Behandlung bestimmter Tumoren wie z. B. Plattenepithelkarzinomen oder Adenokarzinomen der Kopf-Hals-Region, insbesondere der Region des Mundbodens oder auch der Nasennebenhöhlen, die Bestrahlung nicht nur von Teilen, sondern von der gesamten Zunge als Weichgewebe, also auch ihrer Randbereiche, indiziert. In Ruhelage, d. h. in Okklusionsstellung, überragt die Zunge normalerweise die Unterkieferzähne und liegt am harten Gaumen an. Bei einer Bestrahlung ohne Änderung der Zungenposition würden daher die Zähne, insbesondere die Zahnkronen mitbestrahlt, was zu schweren Schäden des Zahnschmelzes und in weiterer Folge sogar zum Verlust von zuvor gesunden Zähne führen kann. Darüber hinaus ist eine so genannte Osteoradionekrose der Kieferknochen - also ein strahlungseffizientes und irreparables Absterben (Nekrose) von Knochensubstanz - insbesondere nach Extraktion eines Zahnes aus dem bestrahlten Knochen beobachtet worden, was mitunter zu einem Verlust großer Kieferanteile und somit, neben den damit unmittelbar verbundenen medizinischen und Alltagsproblemen, zu einer deutlichen Einbuße der Lebensqualität des Patienten führt, also zu einer medizinisch-induzierten physischen ebenso wie einer psychischen Beeinträchtigung. In einer solchen Situation ist das zu behandelnde Gewebe ein Weichgewebe, das zur Behandlung, die aus mehreren Sitzungen bestehen kann, in eine reproduzierbare Position gebracht und in dieser während einer Sitzung gehalten werden muss. Es ist jedoch auch denkbar, dass gesundes Weichgewebe aus dem Wirkungsbereich der Strahlen gehalten bzw. von diesem ferngehalten werden, d. h. dafür Sorge getragen werden muss, dass dieses auch nicht nur vorübergehend den Strahlengang zwischen der Strahlenquelle und dem zu behandelnden Gewebe blockiert. Im Falle von Weichgeweben bei der Teletherapie gilt es somit einen Fall, in dem das Weichgewebe zu seiner eigenen Behandlung in einer wohl definierten und reproduzierbaren Position gehalten werden muss, von einem Fall zu unterscheiden, in dem das Weichgewebe zu seinem Schutz während der Behandlung eines umliegenden Gewebes aus dem Wirkungsbereich der Strahlung gehalten werden muss.

Andere Schutzvorkehrungen sind bei einer Brachytherapie vorzunehmen, die davon abhängen, ob z. B. die Strahlenquelle direkt ins Tumorgewebe eingebracht (interstitielle Brachytherapie) oder direkt neben dem Tumorgewebe angeordnet (Kontaktbrachytherapie) wird, und ob es sich um eine temporäre oder eine permanente Brachytherapie handelt.

Aus WO 2009/091354 ist ein Gewebeprotektor zur Abschirmung gesunden Gewebes bekannt.

Die Dauer einer Strahlenbehandlung (Teletherapie oder temporäre Brachytherapie) hängt von vielen Faktoren wie z. B. von der Art und Lage des Tumors ab. Es können bis zu 30 Sitzungen erforderlich sein, so dass sich die Behandlung über einige Wochen erstrecken kann, wobei die reine Bestrahlungszeit in einer Sitzung ca. 2 bis 3 Minuten und die gesamte Sitzung bis zu 15 Minuten beträgt.

Es ist somit eine Aufgabe der vorliegenden Erfindung, eine Vorrichtung - hierin als Gewebeprotektor bezeichnet - vorzuschlagen, wobei die Vorrichtung zweierlei leisten soll:
1. Sie soll während der Dauer der Strahlenbehandlung eine stabile Bisshebung (d. h. eine vertikale Erhöhung des Abstandes zwischen Ober- und Unterkiefer, auch Nonokklusion genannt) der Kiefer gewährleisten und eine visuelle Kontrolle der Behandlung ermöglichen; und
2. Sie soll dazu geeignet sein, ein Weichgewebe während einer Sitzung in einer Position zu halten, (a) die wohl definiert und reproduzierbar ist, wenn das Weichgewebe selbst behandelt werden soll, und (b) an der das Weichgewebe aus dem Wirkungsbereich der Strahlung gehalten ist, wenn das Weichgewebe während der Behandlung eines umliegenden Gewebes vor der Strahlung geschützt werden soll. Eine Kombination von (a) und (b) ist denkbar. Das heißt, es kann zum Beispiel bei der oben beschriebenen Zungenbehandlung notwendig sein, zusätzlich die Wangen des Patienten aus dem Wirkungsbereich der Strahlen zu halten.

Diese Aufgabe wird durch die Merkmale des Anspruchs 1 gelöst.

Gemäß der vorliegenden Erfindung umfasst ein Gewebeprotektor zum Schutz gesunden Gewebes eines Patienten vor Strahlung bei einer Teletherapie einen Kieferteil zur Erzeugung einer Bisshebung, und einen mit dem Kieferteil verbundenen Weichgewebe-Positionierungsteil zum Positionieren eines Weichgewebes des Patienten, wobei das Kieferteil eine Öffnung zur Sichtkontrolle eines Operationsbereichs des Patienten. Die Lage des Tumors bestimmt die optische Achse der Bestrahlung, die durch die Öffnung führen kann, aber nicht muss, da das Kieferteil zumindest im Frontbereich vorzugsweise aus einem Material gebildet ist, das kein Bestrahlungshindernis darstellt, die verwendete(n) Wellenlänge(n) also nur unwesentlich absorbiert und streut. Die erfindungsgemäß verwendete Strahlung ist vorzugsweise elektromagnetische Mehrfeldstrahlung (Röntgenstrahlen) mit einer Energie in der Größenordnung von 4 - 20 MeV, die tief (bis zu 2 cm) in das Gewebe eindringen kann. Die Angabe von 4 - 20 MeV stellt jedoch nur eine grobe Orientierung dar, der verwendete Wert kann im Einzelfall von diesem Wert abweichen. Der Kieferteil des erfindungsgemäßen Gewebeprotektors erfüllt zwei Aufgaben. Er soll zum einen, gegebenenfalls in Verbindung mit dem Weichgewebe-Positionierungsteil, eine Bisshebung erzeugen und so die Oberkieferzähne aus dem Strahlenfeld rücken. Zum anderen dient er als Befestigungsmittel, um eine exakte Position des Gewebeprotektors, insbesondere des Weichgewebe-Positionierungsteils im Mund reproduzierbar und lagestabil herzustellen.

Gemäß einer vorteilhaften Ausgestaltung der vorliegenden Erfindung ist zwischen dem Kieferteil und dem Weichgewebe-Positionierungsteil eine lösbare Verbindung vorgesehen. Dies hat zum einen den Vorteil, dass beide Teile unabhängig voneinander hergestellt werden können, die Herstellung somit auf unterschiedliche Materialien für den Kiefern und den Weichgewebe-Positionierungsteil angepasst werden kann, und zum anderen den Vorteil, dass dadurch das Einsetzen des erfindungsgemäßen Gewebeprotektors in den Mund des Patienten erleichtert ist, indem beide Teile nacheinander in den Mund eingeführt und in situ miteinander verbunden werden. Alternativ können jedoch, wie es auch in Anspruch 1 der vorliegenden Erfindung in allgemeiner Form zum Ausdruck kommt, der Kieferteil und der Weichgewebe-Positionierungsteil einteilig, als Monoblock ausgebildet sein.

Die Verbindung zwischen dem Kieferteil und dem Weichgewebe-Positionierungsteil ist gemäß einer vorteilhaften Ausgestaltung der vorliegenden Erfindung nach Art eines Matrizen-Patrizen-Systems ausgebildet, wobei beide Teile intraoral durch Ineinanderstecken der einen geeigneten Vorsprung aufweisenden Patrize mit der eine komplementär ausgebildete Vertiefung aufweisenden Matrize eine Presspassung bildend miteinander verbunden werden.

Gemäß einer vorteilhaften Ausgestaltung der vorliegenden Erfindung ist die Matrize im Weichgewebe-Positionierungsteil und die Patrize im Kieferteil ausgebildet. Die umgekehrte Variante ist jedoch äquivalent. Die Matrizen-Patrizen-Verbindung erstreckt sich entlang des Kontaktbereichs zwischen dem Kieferteil und dem Weichgewebe-Positionierungsteil, d. h. im Falle einer zahnbogenförmigen Ausbildung des Kiefer- und Weichgewebe-Positionierungsteils ebenfalls zahnbogenförmig, vorteilhafterweise im Wesentlichen auf deren gesamter Länge von z. B. ca. 40 mm nach Art einer Nut-Feder-Verbindung, wobei in einer zu der Längserstreckung senkrechten Schnittebenenansicht der Kontaktbereich zwischen dem Kiefer- und dem Weichgewebe-Positionierungsteil vorzugsweise als S-förmig gekrümmte Linie erscheint. Die Matrizen-Patrizen-Verbindung kann jedoch auf jede beliebige andere Art ausgebildet sein, solange - wie es in der oben beschriebenen Ausgestaltung der vorliegenden Erfindung der Fall ist - ein Verrutschen, d. h. eine Relativbewegung zwischen dem Kieferteil und dem Weichgewebe-Positionierungsteil während der Behandlung ausgeschlossen ist. Die vorteilhafterweise zahnbogenförmige Ausbildung hat den Vorteil, dass der Druck zur Aufrechterhaltung der Bisshebung vorteilhafterweise auf alle Oberkieferzähne, im Falle eines Unbezahnten auf den gesamten Zahnfleischbogen, gleichmäßig verteilt ist. Darüber kann hierdurch auf einfache Weise eine exakte Position im Mund des Patienten definiert und eine Reproduzierbarkeit dieser Position gewährleistet werden, so dass der Kieferteil während der Sitzung in dieser Position bleibt und bei folgenden Sitzungen wieder in diese Position gebracht werden kann.

Es ist zu beachten, dass durch die hierin gewählte Terminologie, die auf (zahn-) medizinische Lage- und Richtungsbezeichnungen (z. B. "kaudal") sowie die allgemeine Anatomie des Menschen (z. B. "zahnbogenförmig") Bezug nimmt, implizit eine korrekte Position am Menschen in eingesetztem Zustand des Gewebeprotektors bzw. eine an die Anatomie des Menschen angepasste Formgebung definiert ist.

Gemäß der vorliegenden Erfindung ist umfasst das Weichgewebe-Positionierungsteil ein Zungenteil zum Niederhalten der Zunge des Patienten, wobei vorteilhafterweise der Zungenteil einen mit der Patrize verbundenen Zungenniederhalteabschnitt umfasst. Diese Verbindung ist vorteilhafterweise einteilig ausgebildet. Im Hinblick auf die oben beschriebene Zahnproblematik bei teletherapeutischer Behandlung der Zunge kann der Gewebeprotektor gemäß dieser vorteilhaften Ausgestaltung als Gewebeprotektor bezeichnet werden.

Der Zungenniederhalteabschnitt ist gemäß einer vorteilhaften Ausgestaltung der vorliegenden Erfindung im Frontalschnitt im Wesentlichen zungenförmig oder löffelartig, so dass die Zunge durch ihn anatomisch gleichmäßig, insbesondere flächig und nicht punktuell niedergedrückt wird. Der Zungenniederhalteabschnitt kann die Zunge vollständig oder nur teilweise, z. B. nur einen vorderen oder nur einen hinteren Teil, überdecken. Wie es oben schon erwähnt ist, ist der Zungenniederhalteabschnitt vorzugsweise aus einem Material gebildet, das für den Wellenlängenbereich der verwendeten Strahlung im Wesentlichen transparent und nicht streuend ist, wobei die Eigenschaft der Transparenz die Energiemenge bestimmt, die insgesamt durch den erfindungsgemäßen Gewebeprotektor hindurchtritt, und die Eigenschaft möglichst geringer Streuung eine hohe Energiedichte in einem wohl definierten und möglichst kleinen Bestrahlungsbereich ergibt.

Gemäß der vorliegende Erfindung umfasst der Weichgewebe-Positionierungsteil ein Lippenteil, das so bezüglich des Kieferteils angeordnet ist, dass es die Lippen des Patienten während der Behandlung vom Wirkungsbereich der Strahlen fernhält. Erfindungsgemäß umfasst somit das Weichgewebe-Positionierungsteil entweder nur einen Zungenteil oder nur einen Lippenteil, oder sowohl einen Zungenteil als auch einen Lippenteil. Da sich bei appliziertem Gewebeprotektor der Zungenteil überwiegend in der Mundhöhle befindet, ist es möglich oder gar notwendig, dass sich der Lippenteil, insbesondere wenn er als Teil konzipiert und ausgelegt ist, der dem Halten der Oberlippe des Patienten dient, nicht mit dem Zungenteil, sondern mit dem Kieferteil verbunden ist. Die Unterteilung in Weichgewebe-Positionierungsteil und Kieferteil kann daher erfindungsgemäß auch eine rein funktionale sein, wobei der Zungenteil und der Lippenteil als Teile des Weichgewebe-Positionierungsteils nicht einteilig miteinander verbunden sind. Insbesondere kann zwischen dem Zungenteil und dem Kieferteil die oben genannte lösbare Verbindung angeordnet sein, während die Verbindung zwischen dem Kieferteil und dem Lippenteil einteilig sein kann. Ferner kann der Lippenteil aus räumlich getrennten Lippenteilabschnitten gebildet sein. Vorteilhafterweise ist die Verbindung zwischen dem Lippenteil und dem Kieferteil derart, dass der Lippenteil gegenüber dem Kieferteil bei appliziertem Gewebeprotektor verlagerbar ist.

Gemäß der vorliegenden Erfindung umfasst das Weichgewebe-Positionierungsteil einen Wangenteil, der so bezüglich des Kieferteils angeordnet ist, dass es die Wangen des Patienten während der Behandlung vom Wirkungsbereich der Strahlen fernhält. Für den Wangenteil gilt Entsprechendes wie für den Lippenteil. Insbesondere kann der Wangenteil aus mehreren Wangenteilabschnitten bestehen, die weder untereinander noch mit dem Zungenteil oder dem Lippenteil verbunden sind.

Gemäß einer vorteilhaften Ausgestaltung der vorliegenden Erfindung weist der erfindungsgemäße Gewebeprotektor wenigstens einen Durchstrahlungsbereich auf, dessen Material und/oder Wanddicke dazu ausgelegt ist, das Strahlungsmaximum der Strahlung in einen Oberflächenbereich des Gewebes zu verschieben. Auf diese Weise kann die Strahlendosis am Ort des Tumors beeinflusst bzw. optimiert werden. Werden zum Beispiel zur Behandlung Elektronenstrahlen verwendet, so wird aufgrund der geringen Wechselwirkung der Elektronen beim Eintritt in das Gewebe, die dort noch eine sehr hohe kinetische Energie besitzen, mit dem Gewebe ein Großteil dieser Energie in tieferen Gewebeschichten deponiert. Mit anderen Worten: Die Tiefendosiskurve für Elektronen, die die Abhängigkeit der Strahlendosis von der Eindringtiefe zeigt, besitzt bei einer von der kinetischen Energie der Elektronen abhängigen und von Null verschiedenen Tiefe ein Maximum. Die in einem Oberflächenbereich absorbierte Energie ist daher unter Umständen nicht ausreichend, um einen dort befindlichen Tumor zu zerstören. Daher muss die Strahlung vor Eintritt in das Gewebe durch ein so genanntes gewebeäquivalentes Material geleitet und von diesem teilweise absorbiert werden. Durch eine Verdickung der Kunststoffschicht des erfindungsgemäßen Gewebedetektors an einer Stelle oder an mehreren Stellen, die hier als Durchstrahlungsbereich(e) bezeichnet ist (sind), gegenüber den übrigen Wanddicken des Gewebedetektors kann dieses Maximum an den Ort des Tumors gerückt werden. Die Dicke der Kunststoffschicht des erfindungsgemäßen Gewebeprotektors an einem solchen Durchstrahlungsbereich, dessen Lage in appliziertem Zustand des Gewebeprotektors mit der Lage des Tumors korrespondiert, liegt - tumorabhängig - in der Größenordnung von 1 cm. Die Wirkung der "verdickten" Druchstrahlungsbereiche kann mit einem Moderator zur Erzeugung thermischer Neutronen in einem Kernreaktor verglichen werden. Der beschriebene Effekt kann somit, wie es oben beschrieben ist, entweder durch eine geeignete Dicke der Wand im Bereich des Durchstrahlungsbereichs und/oder durch ein geeignetes Material bewirkt werden. Das Material des Durchstrahlungsbereichs kann ferner identisch mit dem Material der übrigen Bereiche des Gewebeprotektors oder von diesem verschieden sein.

Gemäß der vorliegenden Erfindung umfasst ein Gewebeprotektor zum Schutz gesunden Gewebes eines Patienten vor Strahlung bei einer Brachytherapie ein Kieferteil zur Erzeugung einer Bisshebung (ebenso wie bei dem oben beschriebenen Gewebeprotektor zum Schutz gesunden Gewebes eines Patienten vor Strahlung bei einer Teletherapie), wobei der Kieferteil wenigstens einen Applikationskatheter umfasst. Über den wenigstens einen Applikationskatheter, der vorteilhafterweise in einem Abstand von etwa 3 - 5 mm zur Schleimhautoberfläche in den Kieferteil eingearbeitet ist, wird in der Regel ein Iridium-Strahler, in manchen Fällen auch z. B. ein Cobalt-Strahler, der Gamm-Strahlung mit einer Energie von ca. 4 - 25 MeV aussendet, möglichst nah an den Tumor herangeführt. Im Falle von mehr als einem Applikationskatheter sind diese in einem Abstand von etwa 5 mm zueinander angeordnet. Vorteilhafterweise ist der Applikationskatheter als Kanal in den Gewebeprotektor eingearbeitet und wirkt so als Lichtleiter.

Gemäß der vorliegenden Erfindung umfasst der Gewebeprotektor einen Weichgewebe-Positionierungsteil mit den oben im Zusammenhang mit dem Gewebeprotektor für eine Teletherapie definierten Merkmalen. Dies hat den Vorteil, dass auch bei einer Brachytherapie, wenn nicht, wie es normalerweise der Fall ist, die Strahlenquelle direkt ins Tumorgewebe eingebracht oder direkt neben dem Tumorgewebe angeordnet ist, sondern zwischen dem Applikationskatheder und dem zu bestrahlenden Gewebe ein relativ großer Abstand existiert, gesundes Gewebe aus dem Wirkungsbereich des Strahls gehalten werden kann.

Gemäß einer vorteilhaften Ausgestaltung der vorliegenden Erfindung umfasst der Gewebeprotektor wenigstens eine Bleiplatte zum Strahlenschutz des gesunden Gewebes, die vorteilhafterweise austauschbar vorgesehen ist, wobei die Befestigung der wenigstens einen Bleiplatte vorzugsweise nach Art eines Druckknopfsystems erfolgt. Durch die wenigstens eine Bleiplatte ist ein Schutz des gesunden Gewebes, z. B. der Lippen, der Wangenschleimhaut, der Zunge, des harten Gaumens, des Alveolarkamms etc. gewährleistet. Je nach Strahlendosis - die Energie der Strahlen liegt im Kilovoltbereich - beträgt die Dicke der Bleiplatten etwa 2 - 3 mm. Durch die Austauschbarkeit, d. h. insbesondere der Abnehmbarkeit der Bleiplatten ist es möglich, die üblicherweise verwendeten zwei Schutzvorrichtungen auf nur eine zu begrenzen. Denn üblicherweise werden eine erste Vorrichtung aus Kunststoff und ohne Bleiplatten zur Planung der Brachytherapie, und eine zweite Vorrichtung mit Bleitplatten zur Durchführung der Brachytherapie verwendet.

Gemäß einer vorteilhaften Ausgestaltung der vorliegenden Erfindung ist die Öffnung in dem Kieferteil median angeordnet. Alternativ kann die Öffnung aus dieser Position nach distal versetzt sein, um so z. B. die Sicht auf einen eher am seitlichen Zungenrand gelegenen Tumor zu verbessern.

Gemäß einer vorteilhaften Ausgestaltung der vorliegenden Erfindung ist ein kaudaler Rand des Kieferteils durch einen sich von der Öffnung kaudal erstreckenden Spalt zweigeteilt. Durch diese Zweiteilung wird das Sichtfeld des Strahlentherapeuten nach unten erweitert, ohne auf die oben genannten Vorteile verzichten zu müssen. Dies ist insbesondere aufgrund der Position der Zunge vorteilhaft, die während der Behandlung durch den Zungenteil niedergedrückt ist, sich also an einer tieferen Position befindet. Die Breite des Spalts ist ein Kompromiss zwischen Stabilität und Steifigkeit des erfindungsgemäßen Gewebeprotektors einerseits und einem möglichst großen Sichtfeld andererseits, und entspricht gemäß einer vorteilhaften Ausgestaltung der vorliegenden Erfindung der lateralen Erstreckung der unteren Schneidezähne des Patienten; d. h. durch den Kieferteil wird die Unterkieferzahnreihe nur von den Zähnen 38-33 und 48-43 (bezüglich der Zähne wird stets die übliche zahnmedizinische Nummerierung verwendet) eingefasst. Erfindungsgemäß ist der kraniale Rand des Kieferteils stets durchgehend, weist also keinen sich entsprechend von der Öffnung nach kranial erstreckenden Spalt auf. Es werden somit die durch den erfindungsgemäßen Gewebeprotektor alle Zähne 38-33 und 48-43 des Oberkiefers eingefasst, was zur Stabilität und Handhabung des Gewebeprotektors beiträgt.

Gemäß einer vorteilhaften Ausführungsform der vorliegenden Erfindung weist der Gewebeprotektor wenigstens ein Fixierungselement zu seiner Fixierung auf. Diese Fixierung ist insbesondere bei der Strahlentherapie von Kopf- bzw. Halstumoren und auch bei Gehirntumoren erforderlich und erfolgt dadurch, dass das wenigstens eine Fixierelement über ein Befestigungssystem mit einem Bestrahlungstisch oder einem Gesichtsbogen oder dergleichen verbunden wird, so dass eine völlige Immobilisierung und reproduzierbare Positionierung des Gewebeprotektors und somit Kopfes erreicht wird. Auf diese Weise kann zum Beispiel auf eine herkömmliche Kunststoffgipsmaske zur Kopffixierung verzichtet werden. Die Hauptwirkung der Fixierung liegt dabei in der Immobilisierung des Unter- und Oberkiefers durch den erfindungsgemäßen Gewebeprotektor. Das wenigstens eine Fixierelement ist vorteilhafterweise einteilig mit dem Kieferteil verbunden und weist eine gabelförmige Struktur mit zwei Zinken auf. Im Zwischenraum der Zinken ist ein Verbindungsteil angeordnet, das zu einem Gesichtsbogen führt, der wiederum an einer vorzugsweise individuell angefertigten Hinterhauptschale oder an einem Tischhalterungssystem fixiert ist. Zur zusätzlichen Stabilität ist der Gesichtsbogen vorteilhafterweise an der Nasenwurzel, dem Nasion, über eine so genannte Nasionsstütze abgestützt, die ebenfalls vorteilhafterweise individuell angefertigt ist. Zur Unterkieferimmobilisierung ist es weiterhin vorteilhaft, eine Kinnkappe vorzusehen, die ebenfalls an der Hinterhauptschale befestigt ist.

Gemäß einer vorteilhaften Ausgestaltung der vorliegenden Erfindung wird der Schutz von gesundem Gewebe dadurch verbessert, dass das Kieferteil so ausgebildet ist, dass durch das Kieferteil die Zahnkronen von wenigstens einem Teil der Ober- und Unterkieferzähne wenigstens teilweise umschlossen ist. Welche Zahnkronen dies betrifft, hängt u. a. von der Lage des Tumors und von der Zahl der Zähne ab.

Gemäß einer vorteilhaften Ausführungsform umfasst der Gewebedetektor einen Sensor zur Erfassung der Strahlenbelastung. Dies dient zum einen der Beurteilung und Überprüfung, ob die geplante Dosis den Zielort, d. h. den Tumor erreicht. Und zum anderen der Beurteilung, ob die Dosis an anderen Stellen, die geschont werden sollen, überschritten wird oder nicht.

Gemäß einer vorteilhaften Ausführungsform umfasst der Gewebedetektor wenigstens eine Tumormarkierung. Dies hat den Vorteil, dass eine Tätowierung des Tumors auf der Zunge, im Lippen-/Wangenbereich etc., wie es herkömmlich häufig gemacht wird, nicht mehr notwendig ist, da der Tumor an geeigneter Stelle auf dem durchsichtigen Gewebedetektor markiert ist. Die Markierung kann vorteilhafterweise mit einer Kunststoffschicht überdeckt oder versiegelt werden. So ist auch nach der Bestrahlung die ursprüngliche Position und Größe des Tumors ersichtlich. Der Patient erspart sich dadurch eine Vollnarkose mit dem damit verbundenen Aufenthalt in einem Krankenhaus. Die Markierung bietet darüber hinaus den Vorteil, dass der Behandlungsprozess erkennbar ist, wenn die Markierung ursprünglich die gleiche Größe wie der Tumor zu Beginn der Behandlung besitzt.

Gemäß einer vorteilhaften Ausgestaltung der vorliegenden Erfindung ist der Kieferteil und/oder der Weichgewebe-Positionierungsteil aus einem festen Kunststoff gebildet, wobei "fest" hierin bedeutet, dass eine konstante Bisshebung und konstante räumliche Relationen zwischen dem Ober- und dem Unterkiefer - und der Zunge gewährleistet ist. Dies schließt jedoch eine gewisse Formelastizität nicht aus. Im Gegenteil. Um die oben beschriebenen Funktionen optimal erfüllen zu können, ist es vorteilhaft, dass die Zähne die Oberfläche des Kiefer- bzw. Weichgewebe-Positionierungsteil, insbesondere des Zungenteils geringfügig elastisch eindrücken.

Gemäß einer vorteilhaften Ausgestaltung der vorliegenden Erfindung ist der Gewebeprotektor individuell an den Patienten angepasst. Dadurch werden sowohl die Präzision des chirurgischen Eingriffs und die Reproduzierbarkeit der relativen Positionen von Oberkiefer, Unterkiefer und Zunge in den einzelnen Sitzungen, als auch der Tragekomfort des Gewebeprotektors für den Patienten, was zu einer Reduzierung z. B. von Schleimhautirritationen beiträgt, erhöht.

Die obigen und weitere Aufgaben, Eigenschaften und Vorteile der vorliegenden Erfindung sind aus der nachfolgenden detaillierten Beschreibung, die unter Bezugnahme auf die beigefügten Zeichnungen gemacht wurde, deutlicher ersichtlich. In den Zeichnungen sind:
Fig. 1 eine Vorderansicht eines Gewebeprotektor gemäß einer Ausführungsform der vorliegenden Erfindung;
Fign. 2 bis 4 der Gewebeprotektor nach Fig. 1, eingesetzt in einen menschlichen Schädel, in einer Vorderansicht, einer Seitenansicht und einer Rückansicht;
Fign. 5 bis 8 perspektivische Ansichten des Gewebeprotektors gemäß weiteren Ausführungsformen der vorliegenden Erfindung; und
Fig. 9 und Fig. 10 eine Frontal- bzw. Seitenansicht, die den erfindungsgemäßen Gewebeprotektor in Kombination mit einer Vorrichtung zur Fixierung des Gewebeprotektors am Kopf eines Patienten zeigen.

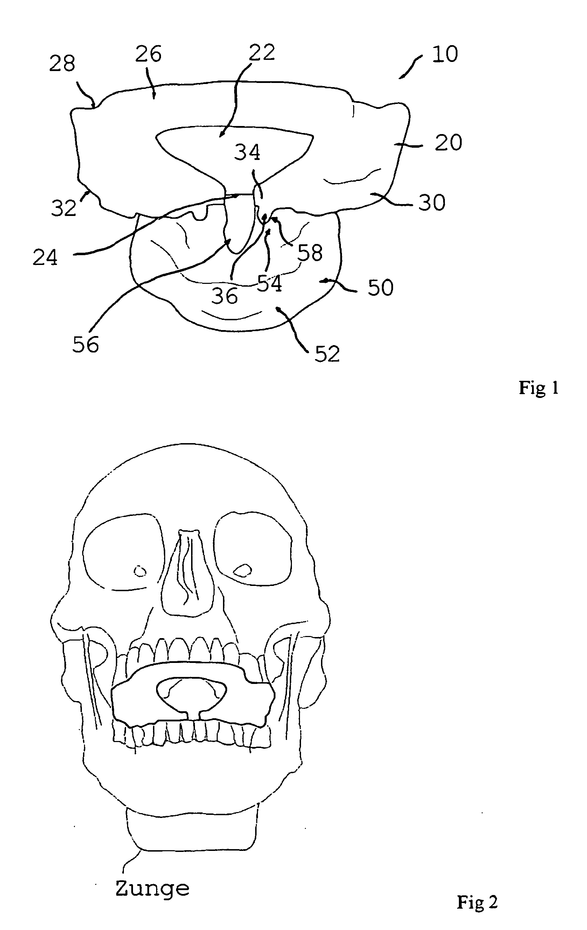

Fig. 1 zeigt eine Vorderansicht eines Gewebeprotektors gemäß einer Ausführungsform der vorliegenden Erfindung, der allgemein mit 10 bezeichnet ist. Der Gewebeprotektor 10 umfasst einen Kieferteil 20 und einen Zungenteil 50.

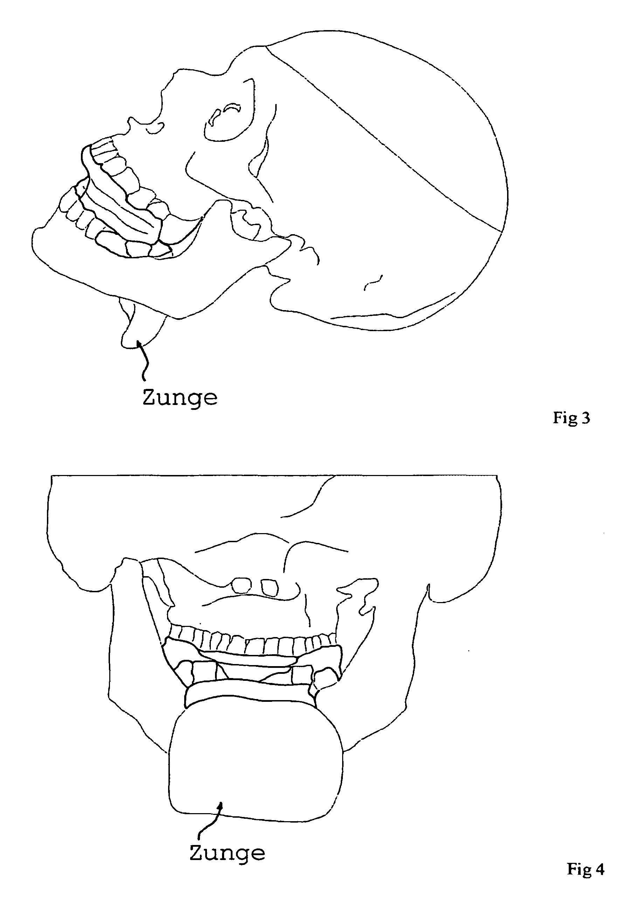

Der Kieferteil 20 ist in seiner Längserstreckung zahnbogenförmig ausgebildet, d. h. er erstreckt sich in eingesetztem Zustand entlang des oberen und unteren Zahnbogens, wie es in den Fign. 2 bis 4 gezeigt ist. Der Kieferteil 20 weist ferner in seinem frontalmedialen Abschnitt eine allgemein dreieckförmige Öffnung 22 auf, die kaudal (in Fig. 1 nach unten) in einen nach unten offenen Spalt 24 übergeht. Der Kieferteil 20 weist einen Oberkieferabschnitt 26 mit einer oberen Zahnauflage- oder Stützfläche 28 und einen mit dem Oberkieferabschnitt 26 einteilig verbundenen Unterkieferabschnitt 30 mit einer unteren Zahnauflage- oder Stützfläche 32 auf. Der Unterkieferabschnitt 30 weist ferner einen sich nach lingual erstreckenden Patrizenabschnitt 34 auf, von dem ein sich ebenfalls entlang der gesamten Länge des Patrizenabschnitts 34 erstreckender Steg 36 kaudal hervorragt. Durch die Öffnung 22 und den Spalt 24 ist der Unterkieferabschnitt 30 einschließlich dessen Patrizenabschnitt 34 in einen linken und einen rechten Abschnitt geteilt. Der Unterkieferabschnitt 30 fasst daher bei der Anwendung an einem vollbezahnten Patienten nur die Unterkieferzähne 38-33 und 48-43 ein; die unteren Schneidezähne sind ausgespart. Der Oberkieferabschnitt 26 hingegen ist durchgehend ausgebildet, so dass er bei der Anwendung an dem vollbezahnten Patienten sämtliche Zähne 18-11 und 28-21 einfasst.

Der Zungenteil 50 umfasst einen Zungenniederhalteabschnitt 52 und einen einteilig mit diesem verbundenen Matrizenabschnitt 54. Der Matrizenabschnitt 54 weist einen vertikalen Spalt 56 auf, durch den er in einen linken und einen rechten Abschnitt geteilt ist. Der Matrizenabschnitt 54 weist ferner eine zu dem Steg 36 des Patrizenabschnitts 34 komplementär ausgebildete Nut 58 auf, in der in einem in den Mund des Patienten eingesetzten Zustand des erfindungsgemäßen Gewebeprotektors 10 der Steg 36 so aufgenommen ist, dass eine lösbare, für die Behandlung jedoch ausreichend stabile Verbindung nach Art einer Matrizen-Patrizen- oder Nut-Feder-Verbindung zwischen dem Kieferteil 20 und dem Zungenteil 50 gebildet ist. Der Zungenniederhalteabschnitt 52 ist an die Wölbung des Zungenrückens angepasst und dazu geeignet, die Zunge an einer wohl definierten und reproduzierbaren Position unterhalb der Unterkieferzähne niederzuhalten. Wie es in Fig. 1 gezeigt ist, setzt sich der Spalt 24 des Oberkieferabschnitts 30 des Kieferteils 20 durch den Matrizenabschnitt 54 in den Zungenniederhalteabschnitt 52 fort. Wie es in Fig. 1 gezeigt ist, ist in einem Querschnitt durch die Matrizen-Patrizen-Verbindung die Kontaktfläche zwischen dem Matrizenabschnitt 54 und dem Patrizenabschnitt 34 als S-förmige Linie sichtbar, die das Ineinandergreifen des Stegs 36 und der Nut 58 zeigt.

Die Fign. 2 bis 4 zeigen den Gewebeprotektor 10 der Fig. 1 in eingesetztem Zustand. Deutlich erkennbar ist die durch den Gewebeprotektor 10 erreichte Bisshebung und Niederdrückung der Zunge, die eine zahnunbedenkliche Bestrahlung unter Sichtkontrolle durch die Öffnung 22 und den Schlitz 24 ermöglicht.

Gemäß der Ausführungsform ist der Gewebeprotektor 10 aus einem festen, aber von den Zähnen oberflächig leicht elastisch eindrückbaren Material gebildet, das nahezu verlustfrei durchstrahlt werden kann. Im Übrigen kann der Gewebeprotektor 10 nach jeder Sitzung einfach, z. B. mit Hilfe einer Zahnbürste und Wasser, gereinigt werden.

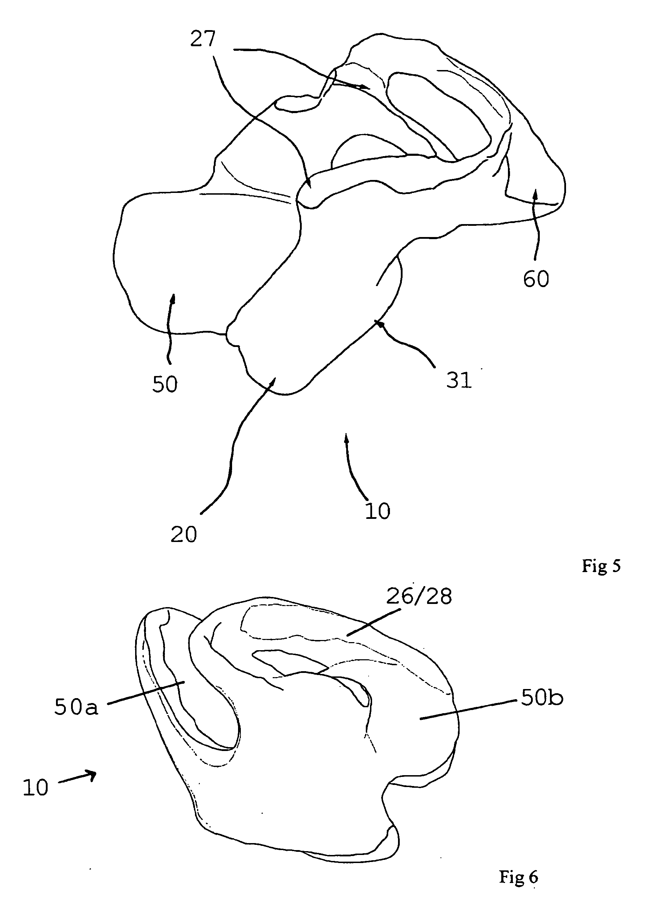

Fig. 5 zeigt eine weitere Ausführungsform des Gewebeprotektors 10 der vorliegenden Erfindung. Der Gewebeprotektor 10 ist als Monoblock ausgebildet, wobei der Kieferteil 20, der eine Oberkieferkauleiste 27 und eine Unterkieferkauleiste 31 umfasst, mit dem Zungenteil 50 einteilig verbunden ist. Der Gewebeprotektor 10 weist ferner ein Lippenteil 60 auf, das einteilig mit dem Kiefer-Zungenteil verbunden ist.

Fig. 6 zeigt eine weitere Ausführungsform des Gewebeprotektors 10 der vorliegenden Erfindung. Der Gewebeprotektor 10 ist wiederum als Monoblock ausgebildet. Nach links hervorragend ist ein vorderer Zungenteilabschnitt 50a des Zungenteils 50, und nach rechts hervorragend ein hinterer Zungenteilabschnitt 50b des Zungenteils 50, die gemeinsam dafür sorgen, dass die Zunge nach kranial-anterior gedrückt wird. Die Bezugszeichen 26 und 28 zeigen wie bei den oben beschriebenen Ausführungsformen den Oberkieferabschnitt bzw. die obere Zahnauflage- oder Stützfläche.

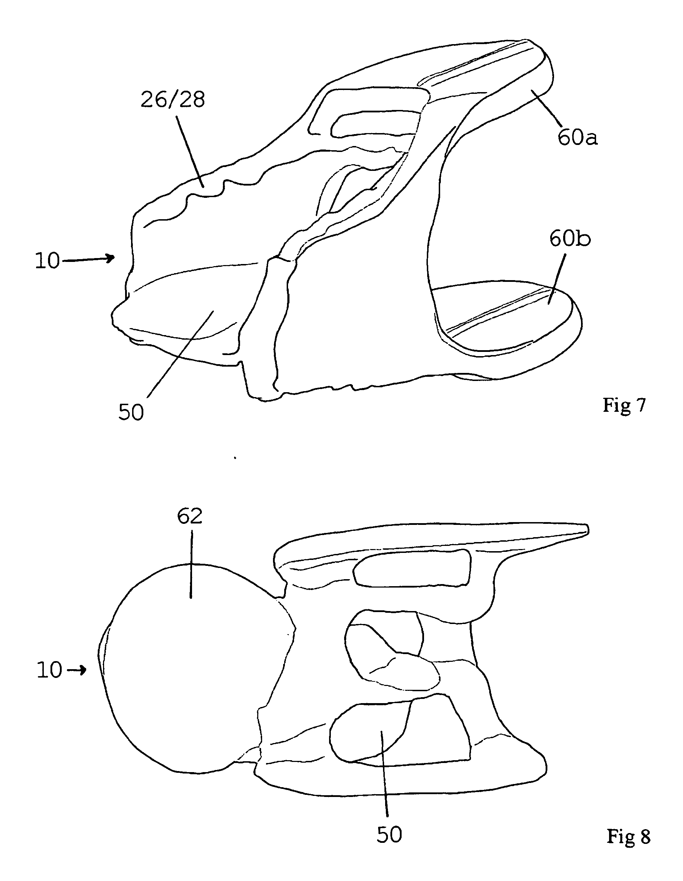

Fig. 7 zeigt eine weitere Ausführungsform des Gewebeprotektors 10 der vorliegenden Erfindung. Deutlich nach rechts hervorragend sind ein oberer Lippenteilabschnitt 60a und ein unterer Lippenteilabschnitt 60b. In der Mitte, zwischen dem Oberkieferabschnitt 26 bzw. der Zahnauflage- oder Stützfläche 28 ist das Zungenteil 50, das die Zunge fixiert, die aufgrund ihres eigenen Muskeltonus von unten gegen das Zungenteil 50 einen Druck ausübt.

Fig. 8 zeigt eine weitere Ausführungsform des Gewebeprotektors 10 der vorliegenden Erfindung. In dieser Ausführungsform ragt ein Wangenteil 62 nach links - bezüglich des Körpers des Patienten nach lateral rechts hervor. Durch Öffnungen in dem Gewebeprotektor 10 ist hinten das Zungenteil 50 zu erkennen, das in dieser Ausgestaltung dazu dient, die Zunge in die gleiche laterale Richtung wegzuhalten wie die das Wangenteil 62 die rechte Wange des Patienten.

Obwohl es in den einzelnen Figuren nicht gezeigt ist, können alle beschriebenen Ausführungsformen im Umfang der Ansprüche und gemäß deren Würdigung in der Beschreibungseinleitung wenigstens einen Durchstrahlungsbereich, dessen Material und/oder Wanddicke dazu ausgelegt ist, das Strahlungsmaximum der Strahlung in einen Oberflächenbereich des Gewebes zu verschieben, wenigstens einen Applikationskatheter, der vorteilhafterweise in einem Abstand von etwa 3 - 5 mm zur Schleimhautoberfläche in den Kieferteil eingearbeitet ist und über den z. B. ein Iridium-Strahler möglichst nah an den Tumor herangeführt, und/oder wenigstens eine Bleiplatte zum Strahlenschutz des gesunden Gewebes, die vorteilhafterweise austauschbar vorgesehen ist, wobei die Befestigung der wenigstens einen Bleiplatte vorzugsweise nach Art eines Druckknopfsystems erfolgt umfassen.

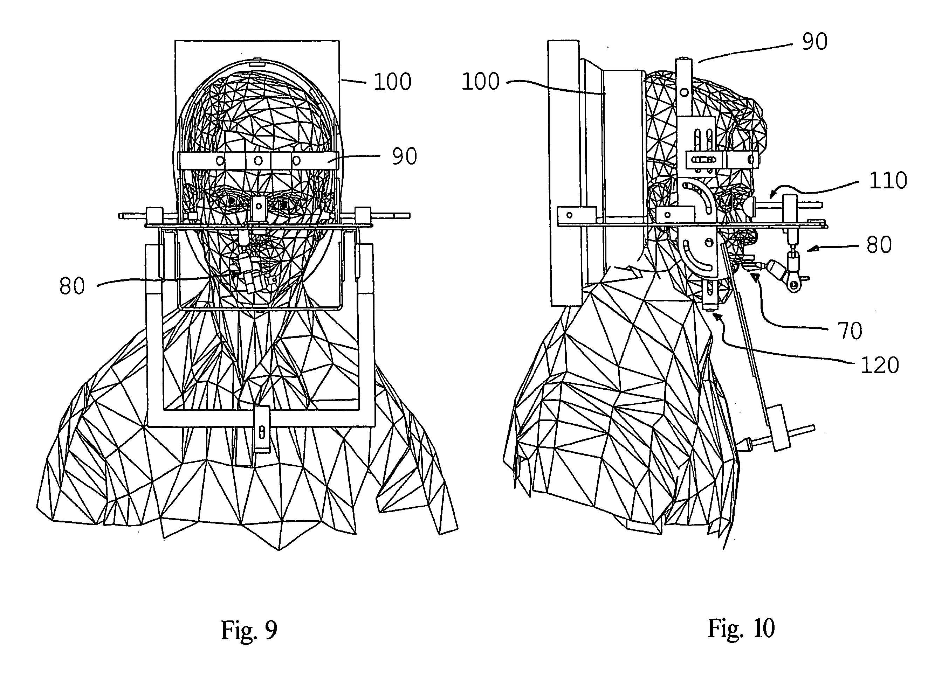

Fign. 9 und 10 den erfindungsgemäßen Gewebeprotektor in Kombination mit einer Vorrichtung zur Fixierung des Gewebeprotektors am Kopf eines Patienten. Eine Fixierung ist insbesondere bei der Strahlentherapie von Kopf- bzw. Halstumoren und auch bei Gehirntumoren erforderlich. Der Gewebeprotektor weist hierzu ein Fixierungselement 70 auf, das über ein Befestigungssystem 80 mit einem (nicht gezeigten) Bestrahlungstisch oder einem an sich bekannten Gesichtsbogen 90 oder dergleichen verbunden ist, so dass eine völlige Immobilisierung und reproduzierbare Positionierung des Gewebeprotektors relativ zum Kopf des Patienten erreicht wird. Auf diese Weise kann zum Beispiel auf eine herkömmliche Kunststoffgipsmaske zur Kopffixierung verzichtet werden. Die Hauptwirkung der Fixierung liegt dabei in der Immobilisierung des Unter- und Oberkiefers durch den erfindungsgemäßen Gewebeprotektor. Das Fixierelement 70 ist vorteilhafterweise einteilig mit dem Kieferteil 20 verbunden und weist im Wesentlichen eine vom Kieferteil 20 weg nach außen ragende gabelförmige Struktur mit zwei Zinken auf.

Im Zwischenraum der Zinken ist ein Verbindungsteil angeordnet, das zu dem Gesichtsbogen 90 führt, der wiederum an einer vorzugsweise individuell angefertigten Hinterhauptschale 100 oder an dem Tischhalterungssystem fixiert ist. Zur zusätzlichen Stabilität ist der Gesichtsbogen 100, wie in Fign. 9 und 10 gezeigt, vorteilhafterweise an der Nasenwurzel, dem Nasion, über eine so genannte Nasionsstütze 110 abgestützt, die ebenfalls vorteilhafterweise individuell angefertigt ist. Zur Unterkieferimmobilisierung ist es weiterhin vorteilhaft, wie in Fig. 9 und 10 gezeigt, eine Kinnkappe 120 vorzusehen, die ebenfalls an der Hinterhauptschale 100 befestigt ist.

Es sei an dieser Stelle darauf hingewiesen, dass der Kieferteil 20 per se, d.h. der Kieferteil 20 ohne die weiteren Elemente des erfindungsgemäßen Gewebeprotektors, zusammen mit der in Fign. 9 und 10 gezeigten Vorrichtung ein eigenständiges System zur Fixierung bzw. Ruhigstellung des Kopfs eines Patienten zum Zweck z.B. einer Strahlentherapie darstellt, für das zu einem späteren Zeitpunkt z.B. im Wege einer Teilanmeldung um Schutz nachgesucht werden kann.

Der Gewebeprotektor 10 gemäß der Ausführungsform kann zum Beispiel mit einem Verfahren hergestellt werden, das allgemein die folgenden Schritte umfasst:
- Herstellen von Abdrücken des Oberkiefers, des Unterkiefers und der Zunge des Patienten;
- Herstellen von Gipsmodellen des Oberkiefers, des Unterkiefers und Zunge des Patienten anhand der Abdrücke;
- Gelenkiges Verbinden des Oberkiefer- und Unterkiefermodells und Einstellen einer wohl definierten Bisshebung zur Herstellung eines Bisshebungsmodells;
- Herstellen des Kieferteils anhand des Bisshebungsmodells; und
- Herstellen des Zungenteils.

Hierbei kann die Herstellung des Oberkieferabdrucks und des Zungenabdrucks gleichzeitig erfolgen. Ferner kann zur Herstellung des Oberkieferabdrucks und des Zungenabdrucks ein Oberkieferabformlöffel verwendet werden, auf dessen Oberseite der Oberkiefer und auf dessen Unterseite die Zunge abgeformt wird. Das Kieferteil kann vorteilhafterweise mittels Kunststoffstreutechnik hergestellt werden. Vorteilhafterweise kann in dem Schritt der Herstellung des Kieferteils der Schritt der Einpolymerisierung der Patrize enthalten sein. Zur Herstellung des Zungenteils ist es ferner vorteilhaft, dass das Gipsmodell der Zunge so in das Gipsmodell des Unterkiefers eingelegt wird, dass das Gipsmodell der Zunge unterhalb der Zahnkronen des Gipsmodells des Unterkiefers angeordnet ist, und der Zungenteil auf dem Zungenrücken des Gipsmodells der Zunge aufpolymerisiert wird.

Konkret kann das Verfahren zur Herstellung des erfindungsgemäßen Gewebeprotektors zum Beispiel wie folgt durchgeführt werden:
Zuerst werden Alginatabdrücke vom Ober- und Unterkiefer beim Patienten genommen. Hierzu wird das Abdruckmaterial mit exakt 20°C warmem Wasser angerührt. Anschließend wird der Abdrucklöffel, der aus Weichplastik besteht, mit dem Material in den Mund des Patienten eingebracht und dort für 10 Minuten belassen. Nach Entnahme muss mindestens eine Stunde die Rückstellung abgewartet werden bevor die Abdrücke ausgegossen werden können.

Für das Ausgießen der Modelle wird Alabastergips verwendet, der im Mischungsverhältnis 200g Gips / 75ml Wasser, 30 Grad warm, angerührt wird.

Die Bissnahme erfolgt beim Zahnlosen unter Zuhilfenahme eventuell vorhandener Prothesen, sonst mit eigens aus lichtgehärtetem Kunststoff angefertigten Bissschablonen mittels Pfeilregistrat nach Gerber. Beim Bezahnten werden zwei 1,5 cm lange Wachsstreifen im Molarenbereich als Aufbissregistrat verwendet.

Nach Erstellen des Registrats in zentrischer Position muss dieses durch nochmalige Bissnahme überprüft werden. Insgesamt müssen fünf Versuche in zentrischer Position reproduzierbar sein. Danach wird die Position des Oberkiefers mittels Gesichtsbogen in einen Artikulator, einem Gerät zur Simulation von Kiefergelenksbewegungen, transferiert.

Im Anschluss daran werden die Gipsmodelle im Artikulator montiert, hierzu wird mit 3%iger Bikarbonatlösung versetztes Wasser zum Befestigen der Gipsmodelle genommen.

Zur Herstellung des Gerätes wird smaragdblauer, durchsichtiger Kunststoff mit eingearbeiteten Lufteinschlüssen verwendet. Die Korngrösse beträgt 100-110 Mikrometer. Nach dem Anrühren muss eine Ansteigzeit von genau 60 Minuten vergehen, bevor der Kunststoff weiterverarbeitet werden kann. Im nächsten Schritt werden die Lufteinschlüsse mittels Druckluft eingebracht. Danach wird der Kunststoff unmittelbar ohne Isoliermittel auf das Gipsmodell im Artikulator aufgebracht und mittels lanzettförmigem Nickel-Titanspatel modelliert, wobei darauf zu achten ist, dass auf dem Oberkiefermodell eine genau 5 mm breite Furche entlang des Kieferkammes entsteht, die nicht tiefer als 5 mm sein darf. Zuvor wurde der Incisalstift um 45 mm erhöht.

Die anschließende Ausarbeitung erfolgt aufgrund des o.a. Kunststoffes mittels 5 µ kreuzverzahnten Hartmetallfräsen in absteigendem Durchmesser des Arbeitsteils bei einer Drehzahl von 80000U/min.

Eine anschließende Politur erfolgt mittels kanarischem Vulkanbimsstein für mindestens 20 Minuten ohne Wasserkühlung, um eine bessere Endpolymerisation zu erreichen. Danach erfolgt noch eine 31 Minuten dauernde Nachpolitur mittels 80%iger Schlämmkreide.

Zum Abschluss des Herstellungsprozesses erfolgt eine Kontrolle. Hierzu wird unter dem Elektronenmikroskop unter 100000-facher Vergrößerung die Ausrichtung der Kunststoffkugeln überprüft, um eine Strahlenstreuung beurteilen zu können.

Schließlich wird das Gerät am Patienten angepasst, d. h. es werden die Bisshöhe, die zentrische Relation und eventuell vorhandene Druckstellen erfasst und entsprechende Korrekturen durchgeführt.

Obgleich die vorliegende Erfindung bezüglich der bevorzugten Ausführungsformen offenbart worden ist, um ein besseres Verständnis von diesen zu ermöglichen, sollte wahrgenommen werden, dass die Erfindung auf verschiedene Weisen verwirklicht werden kann, ohne den Umfang der Erfindung zu verlassen. Deshalb sollte die Erfindung derart verstanden werden, dass sie alle möglichen Ausführungsformen und Ausgestaltungen zu den gezeigten Ausführungsformen beinhaltet, die realisiert werden können, ohne den Umfang der Erfindung zu verlassen, wie er in den beigefügten Ansprüchen dargelegt ist.

### Bezugszeichenliste

10 Gewebeprotektor
20 Kieferteil
22 Öffnung in 20
24 Spalt in 20
26 Oberkieferabschnitt
27 Oberkieferkaufleiste
28 Obere Zahnauflage- oder Stützfläche
30 Unterkieferabschnitt
31 Unterkieferkaufleiste
32 Untere Zahnauflage- oder Stützfläche
34 Patrizenabschnitt
36 Steg an 34
50 Zungenteil
50a vordere Zungenteilabschnitt
50b hinterer Zungenteilabschnitt
52 Zungenniederhalteabschnitt
54 Matrizenabschnitt
56 Spalt
58 Nut in 54
60 Lippenteil
60a oberer Lippenteilabschnitt
60b unterer Lippenteilabschnitt
62 Wangenteil

## Patentansprüche

1. Gewebeprotektor (10) zum Schutz gesunden Gewebes eines Patienten vor Strahlung bei einer Tele- oder Brachytherapie, mit einem Kieferteil (20) zur Erzeugung einer Bisshebung und einem mit dem Kieferteil (20) verbundenen Weichgewebe-Positionierungsteil zum Positionieren eines Weichgewebes des Patienten, wobei das Kieferteil (20) eine Öffnung (22) zur Sichtkontrolle eines Operationsbereichs des Patienten und das Weichgewebe-Positionierungsteil ein Zungenteil (50), ein Lippenteil (60), und / oder ein Wangenteil (62) umfasst, wobei das Zungenteil (50) dem Niederhalten der Zunge des Patienten dient, das Lippenteil (60) so bezüglich des Kieferteils (20) angeordnet ist, dass sich die Lippen des Patienten während der Behandlung nicht im Wirkungsbereich der Strahlen befinden, und [das Wangenteil (62) so bezüglich des Kieferteils (20) angeordnet ist, dass sich die Wangen des Patienten während der Behandlung nicht im Wirkungsbereich der Strahlen befinden.

2. Gewebeprotektor (10) nach Anspruch 1, **dadurch gekennzeichnet, dass** zwischen dem Kieferteil (20) und dem Weichgewebe-Positionierungsteil eine lösbare Verbindung vorgesehen ist.

3. Gewebeprotektor (10) nach Anspruch 2, **dadurch gekennzeichnet, dass** die lösbare Verbindung nach Art eines Matrizen-Patrizen-Systems (34, 54) ausgebildet ist.

4. Gewebeprotektor (10) nach Anspruch 3, **dadurch gekennzeichnet, dass** die Matrize (54) im Weichgewebe-Positionierungsteil ausgebildet ist und eine Längsrichtung aufweist, die zahnbogenförmig gekrümmt ist und entlang der eine in einem zu der Längsrichtung senkrechten Querschnitt S-förmige Nut ausgebildet ist, und dass die Patrize (34) im Kieferteil (20) ausgebildet ist und einen zu der S-bogenförmigen Nut komplementär ausgebildeten Steg (36) aufweist.

5. Gewebeprotektor (10) nach Anspruch 1, **dadurch gekennzeichnet, dass** der Zungenteil (50) einen mit der Patrize (34) verbundenen Zungenniederhalteabschnitt (52) umfasst.

6. Gewebeprotektor (10) nach Anspruch 5, **dadurch gekennzeichnet, dass** der Zungenniederhalteabschnitt (52) einen im Wesentlichen zungenförmigen Frontalschnitt aufweist.

7. Gewebeprotektor (10) nach einem der Ansprüche 1 bis 6, **dadurch gekennzeichnet, dass** er wenigstens einen Durchstrahlungsbereich aufweist, dessen Material und/oder Wanddicke dazu ausgelegt ist, das Strahlungsmaximum der Strahlung in einen Oberflächenbereich des Gewebes zu verschieben.

8. Gewebeprotektor (10) nach einem der Ansprüche 1 bis 7, , **dadurch gekennzeichnet, dass** der Kieferteil (20) wenigstens einen Applikationskatheter umfasst.

9. Gewebeprotektor (10) nach einem der Ansprüche 1 bis 8, **dadurch gekennzeichnet, dass** die Öffnung (22) median angeordnet ist.

10. Gewebeprotektor (10) nach einem der Ansprüche 1 bis 9, **dadurch gekennzeichnet, dass** ein kaudaler Rand des Kieferteils (20) durch einen sich von der Öffnung (22) erstreckenden Spalt (56) zweigeteilt ist.

11. Gewebeprotektor (10) nach Anspruch 10, **dadurch gekennzeichnet, dass** die Breite des Spalts (56) der lateralen Erstreckung der unteren Schneidezähne des Patienten entspricht.

12. Gewebeprotektor (10) nach einem der Ansprüche 1 bis 11, **dadurch gekennzeichnet, dass** das Kieferteil (20) so ausgebildet ist, dass durch das Kieferteil (20) die Zahnkronen von wenigstens einem Teil der Ober- und Unterkieferzähne wenigstens teilweise umschließbar sind.

13. Gewebeprotektor (10) nach einem der Ansprüche 1 bis 12, **dadurch gekennzeichnet, dass** er wenigstens ein Fixierungselement zu seiner Fixierung aufweist.

14. Gewebeprotektor (10) nach einem der Ansprüche 1 bis 13, **dadurch gekennzeichnet, dass** er wenigstens einen Sensor zur Erfassung der Strahlungsbelastung umfasst.

15. Gewebeprotektor (10) nach einem der Ansprüche 1 bis 14, **dadurch gekennzeichnet, dass** er wenigstens eine Tumormarkierung aufweist.

## Claims

1. Tissue protector (10) for the protection of the patient's healthy tissue from radiation exposure during teletherapy or brachytherapy, with a jaw part (20) for bite elevation and a soft tissue positioning part attached to the jaw part (20) for the positioning of the patient's soft tissue; jaw part (20) with an opening (22) for visual control of the treatment area; soft tissue positioning part with a tongue part (50), a lip part (60) and/or a cheek part (62); the tongue part (50) designed to hold down the tongue of the patient; the lip part (60) positioned relative to the jaw part (20) in such a way that the lips of the patient are kept outside the area exposed to radiation; the cheek part (62) positioned relative to the jaw part (20) in such a way that the cheeks of the patient are kept outside the area exposed to radiation.

2. Tissue protector (10) according to claim 1, **characterised by** a detachable connection between the jaw part (20) and the soft tissue positioning part.

3. Tissue protector (10) according to claim 2, **characterised in that** the detachable connection is designed as a female -male connection system (34, 54).

4. Tissue protector (10) according to claim 3, **characterised in that** said female connection element (54) forms part of the soft tissue positioning part, with a longitudinal section following the dental arch, said section featuring an S-shaped groove in a plane orthogonal to said longitudinal section, and **in that** said male connection element (34) forming part of the jaw part (20) has a longitudinal protrusion (36) that matches said S-shaped groove.

5. Tissue protector (10) according to claim 1, **characterised in that** the tongue part (50) includes a tongue depressor section (52) joined to the male connection element (34).

6. Tissue protector (10) according to claim 5, **characterised in that** the front part of the tongue depressor section (52) is essentially shaped as a tongue.

7. Tissue protector (10) according to one of the claims 1 to 6, **characterised in that** it provides at least one radiation section made of materials and/or with a wall thickness designed to shift the maximum radiation exposure to the surface of the tissue.

8. Tissue protector (10) according to one of the claims 1 to 7, **characterised in that** the jaw part (20) comprises at least one application catheter.

9. Tissue protector (10) according to one of the claims 1 to 8, **characterised in that** the opening (22) is arranged at the median.

10. Tissue protector (10) according to one of the claims 1 to 9, **characterised in that** a caudal rim of the jaw part (20) is bisected by a split (56) extending from the opening (22).

11. Tissue protector (10) according to claim 10, **characterised in that** the width of the split (56) corresponds to the lateral extension of the lower incisors of the patient.

12. Tissue protector (10) according to one of the claims 1 to 11, **characterised in that** the jaw part (20) is configured so as to at least partly enclose the crowns of at least some of the upper and lower teeth of the patient.

13. Tissue protector (10) according to one of the claims 1 to 12, **characterised in that** it comprises at least one fixation element for its fixture.

14. Tissue protector (10) according to one of the claims 1 to 13, **characterised in that** it comprises at least one radiation exposure sensor.

15. Tissue protector (10) according to one of the claims 1 to 14, **characterised in that** it comprises at least one tumour marking.

## Revendications

1. Protecteur de tissu (10) pour la protection de tissu sain d'un patient avant irradiation lors d'une téléthérapie ou d'une brachythérapie, avec une partie de mâchoire (20) pour réaliser un soulèvement d'occlusion et une partie de positionnement de tissu mou liée à la partie de mâchoire (20) pour le positionnement d'un tissu mou du patient, la partie de mâchoire (20) comprenant une ouverture (22) pour le contrôle visuel d'une zone d'opération du patient et la partie de positionnement de tissu mou comprenant une partie de langue (50), une partie de lèvres (60) et/ou une partie de joue (62), la partie de langue (50) servant à maintenir la langue du patient vers le bas, la partie de lèvres (60) arrangée par rapport à la partie de mâchoire (20) de manière que les lèvres du patient ne se trouvent pas dans la zone d'action des rayons lors du traitement et la partie de joue (62) arrangée par rapport à la partie de mâchoire (20) de manière que les joues du patient ne se trouvent pas dans la zone d'action des rayons lors du traitement.

2. Protecteur de tissu (10) selon la revendication 1, **caractérisé en ce qu'**une liaison amovible est prévue entre la partie de mâchoire (20) et la partie de positionnement de tissu mou.

3. Protecteur de tissu (10) selon la revendication 2, **caractérisé en ce que** la liaison amovible est formée à la manière d'un système de matrices et patrices (34, 54).

4. Protecteur de tissu (10) selon la revendication 3, **caractérisé en ce que** la matrice (54) dans la partie de positionnement de tissu mou est développée ayant une direction longitudinale qui est courbée dans la forme du secteur denté et le long de laquelle une rainure en forme de S est formée dans une section transversale verticale par rapport à la direction longitudinale, et **caractérisé en ce que** la patrice (34) est formée dans la partie de mâchoire (20) et est munie d'une traverse (36) formée de manière complémentaire par rapport à la rainure en forme d'arc en S.

5. Protecteur de tissu (10) selon la revendication 1, **caractérisé en ce que** la partie de langue (50) comprend une section de dispositif de maintien de langue (52) liée à la partie mâle (34).

6. Protecteur de tissu (10) selon la revendication 5, **caractérisé en ce que** la section de dispositif de maintien de langue vers le bas (52) présente une coupe frontale essentiellement en forme de langue.

7. Protecteur de tissu (10) selon l'une des revendications 1 à 6, **caractérisé en ce qu'**il comporte au moins une zone d'irradiation dont le matériau et/ou l'épaisseur de paroi est adapté(e) pour déplacer le rayonnement maximal de l'irradiation dans une zone superficielle du tissu.

8. Protecteur de tissu (10) selon l'une des revendications 1 à 7, **caractérisé en ce que** la partie de mâchoire (20) comporte au moins un cathéter d'administration.

9. Protecteur de tissu (10) selon l'une des revendications 1 à 8, **caractérisé en ce que** l'ouverture (22) est arrangée de façon médiane.

10. Protecteur de tissu (10) selon l'une des revendications 1 à 9, **caractérisé en ce qu'**un bord caudal de la partie de mâchoire (20) est divisé en deux par une fente (56) s'étendant à partir de l'ouverture (22).

11. Protecteur de tissu (10) selon la revendication 10, **caractérisé en ce que** la largeur de la fente (56) correspond à l'étendue latérale des incisives inférieures du patient.

12. Protecteur de tissu (10) selon l'une des revendications 1 à 11, **caractérisé en ce que** la partie de mâchoire (20) est formée de manière que, par la partie de mâchoire (20), les couronnes dentaires sont au moins en partie entourées par au moins une partie des dents supérieures et inférieures.

13. Protecteur de tissu (10) selon l'une des revendications 1 à 12, **caractérisé en ce qu'**il comporte au moins un élément de fixation pour être fixé.

14. Protecteur de tissu (10) selon l'une des revendications 1 à 13, **caractérisé en ce qu'**il comporte au moins un capteur pour la détection de la charge de rayonnement.

15. Protecteur de tissu (10) selon l'une des revendications 1 à 14, **caractérisé en ce qu'**il comporte au moins un marquage tumoral
